(19) Europäisches Patentamt
European Patent Office
Office européen des brevets

(11) EP 2 778 187 A1

(12) EUROPEAN PATENT APPLICATION
published in accordance with Art. 153(4) EPC

(43) Date of publication:
17.09.2014 Bulletin 2014/38

(51) Int Cl.:
C08G 65/28 (2006.01)     C08G 65/336 (2006.01)
C09K 3/10 (2006.01)

(21) Application number: 12846700.8

(22) Date of filing: 01.11.2012

(86) International application number:
PCT/JP2012/078388

(87) International publication number:
WO 2013/065802 (10.05.2013 Gazette 2013/19)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 04.11.2011  JP 2011241910

(71) Applicant: Asahi Glass Company, Limited
Tokyo 100-8405 (JP)

(72) Inventors:
• ARAI, Takeaki
  Tokyo 100-8405 (JP)
• SUZUKI, Tomoyuki
  Tokyo 100-8405 (JP)
• SUZUKI, Chitoshi
  Tokyo 100-8405 (JP)
• TANAKA, Hideaki
  Tokyo 100-8405 (JP)

(74) Representative: Blodig, Wolfgang
Wächtershäuser & Hartz
Patentanwaltspartnerschaft mbB
Weinstrasse 8
80333 München (DE)

(54) **POLYETHER PREPARATION METHOD, PREPOLYMER PREPARATION METHOD, AND MODIFIED SILICONE POLYMER PREPARATION METHOD**

(57)     It is made possible to prepare a polyether having a high molecular weight with a lower viscosity. A polyether preparation method which comprises a polymerization step of subjecting a monoepoxide having at least 2 carbon atoms to ring-opening addition polymerization to an initiator having at least one active hydrogen-containing functional group in the presence of a catalyst in a stirring vessel 1, to obtain a polyether, wherein the stirring vessel 1 is a stirring vessel wherein a stirring shaft 2 rotatable by an external drive source is provided at the center of the stirring vessel 1; plate-shaped bottom paddles 5, 5 extending in a radial direction of the stirring vessel 1 are mounted on a lower portion of the stirring shaft 2; lattice vanes 6 each comprising arm paddles 7a, 7b, 7c extending in a radial direction and strips 8a, 8b extending in an axial direction, are mounted on a portion of the stirring shaft 2 above the bottom paddles 5, 5; and a discharge nozzle 10 as a monoepoxide-supply means for discharging the monoepoxide to at least two locations below the lower ends of the strips 8a, 8b in the stirring vessel 1.

Fig. 1

EP 2 778 187 A1

**Description**

TECHNICAL FIELD

The present invention relates to a polyether preparation method, a prepolymer preparation method and a modified silicone preparation method.

BACKGROUND ART

Polyethers are widely used as raw materials for polyols for polyurethanes, or sealant materials.

In the preparation of polyethers, it is common to use an alkali catalyst such as KOH, but it is thereby likely that by-products will be formed in an amount exceeding a certain level, and it is difficult to prepare a high molecular weight product having a molecular weight of more than 10,000. Therefore, a high molecular weight polyether is prepared by using a double metal cyanide complex catalyst (hereinafter sometimes referred to as a "DMC catalyst") whereby side-reactions are less likely to occur.

A polyether is usually prepared by a method wherein an initiator and a catalyst are charged into a reactor, and a monoepoxide as a monomer is gradually supplied thereto and subjected to ring-opening addition polymerization. However, as the molecular weight increases, the polyether tends to become highly viscous, and the dispersibility of the monoepoxide supplied gradually during the preparation tends to be lowered, thus leading to a drawback that uniform addition tends to be difficult. In order to overcome such a drawback, a method for improving the dispersibility by using large-sized vanes has been proposed (Patent Document 1).

PRIOR ART DOCUMENT

PATENT DOCUMENT

Patent Document 1: JP-A-5-247199

DISCLOSURE OF INVENTION

TECHNICAL PROBLEM

However, even if a high molecular weight polyether is prepared by the method disclosed in Patent Document 1, the obtained polyether may sometimes be not suitable for use, since its viscosity is high.

The present invention is to provide a polyether preparation method whereby a polyether having a high molecular weight can be prepared with a lower viscosity, and to further provide a prepolymer preparation method and a modified silicone polymer preparation method employing such a polyether.

SOLUTION TO PROBLEM

The present invention provides a polyether preparation method which comprises subjecting a monoepoxide having at least 2 carbon atoms to ring-opening addition polymerization to an initiator having at least one active hydrogen-containing functional group in the presence of a catalyst in a stirring vessel, to obtain a polyether, wherein the stirring vessel is a stirring vessel wherein a stirring shaft rotatable by an external drive source is provided at the center of the stirring vessel; plate-shaped bottom paddles extending in a radial direction of the stirring vessel are mounted on a lower portion of the stirring shaft; lattice vanes each comprising arm paddles extending in a radial direction and strips extending in an axial direction, are mounted on a portion of the stirring shaft above the bottom paddles; and a monoepoxide-supply means for discharging the monoepoxide to at least two locations below the lower ends of the strips in the stirring vessel.

The polyether preferably has a number average molecular weight of at least 10,000.

The catalyst is preferably a double metal cyanide complex catalyst.

It is preferred that the monoepoxide-supply means has discharge openings for discharging the monoepoxide, and the discharge openings are provided below the position of the lower ends of the strips of the lattice vanes.

It is preferred that the discharge openings are present between the bottom paddles and the bottom of the stirring vessel, and the monoepoxide-supply means is provided so as not to be in contact with both of the bottom paddles and the bottom of the stirring vessel.

The monoepoxide-supply means is preferably provided with a ring-shaped discharge nozzle.

It is preferred that into the stirring vessel, the initiator is filled in such an amount that the liquid surface of the initiator becomes higher than the position of the discharge openings for discharging the monoepoxide, and then, the monoepoxide is supplied into the liquid and subjected to the ring-opening addition polymerization.

It is preferred that after filling the double metal cyanide complex catalyst and the initiator into the stirring vessel, a part of the monoepoxide is supplied to activate the double metal cyanide complex catalyst, and after activation of the double metal cyanide complex catalyst, the rest of the monoepoxide is supplied and subjected to the ring-opening addition polymerization.

The amount of the monoepoxide supplied to activate the double metal cyanide complex catalyst is preferably from 3 to 20 mass% to the initiator.

The supply rate of the monoepoxide supplied into the stirring vessel after activation of the double metal cyanide complex catalyst is preferably from 5 to 30 mass%/hr to the amount of the polyether to be prepared.

The present invention provides a modified silicone polymer preparation method which comprises a step of preparing a polyether by the preparation method of the present invention and a step of introducing a hydrolysable silyl group to a molecular terminal of the polyether.

The present invention provides a prepolymer preparation method which comprises a step of preparing a polyether by the preparation method of the present invention and a step of reacting the polyether and a polyisocyanate compound to obtain a prepolymer having an isocyanate group at the terminal.

The present invention provides a modified silicone polymer preparation method which comprises a step of preparing a prepolymer by the preparation method of the present invention and a step of introducing a hydrolysable silyl group to the molecular terminal of the prepolymer.

The modified silicone polymer is preferably a sealant.

ADVANTAGEOUS EFFECTS OF INVENTION

According to the present invention, it is possible to prepare a polyether having a high molecular weight with a lower viscosity by preventing the viscosity from becoming high during the preparation of the polyether.

By using the polyether prepared by the present invention as a raw material, it is possible to prepare a prepolymer or modified silicone polymer having a high molecular weight with a lower viscosity.

BRIEF DESCRIPTION OF DRAWINGS

Fig. 1 is a partially cutaway perspective view illustrating one embodiment of the stirring vessel according to the present invention.
Fig. 2 is a partially cutaway perspective view of a stirring vessel used in Reference Example 1.
Fig. 3 is a partially cutaway perspective view of a stirring vessel used in Reference Example 3.

DESCRIPTION OF EMBODIMENTS

[Stirring vessel]

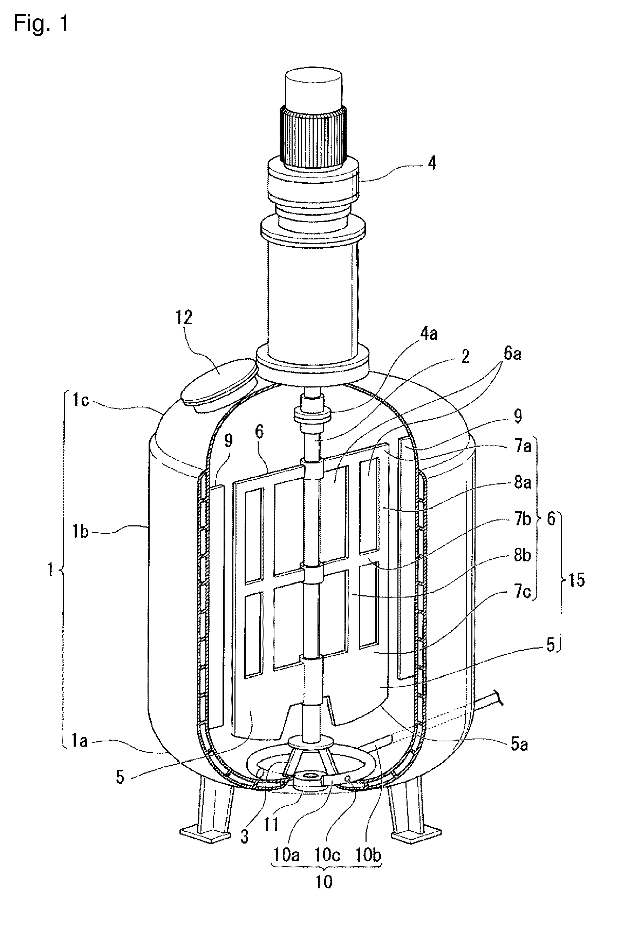

Fig. 1 is a partially cutaway perspective view illustrating an embodiment of the stirring vessel to be suitably used for the preparation method of the present invention.

In the Fig., reference symbol 1 represents a stirring vessel. The stirring vessel 1 has a vertical type substantially cylindrical shape with its central axis direction being in the vertical direction and with its radial direction being in the horizontal direction, and a stirring shaft 2 is provided along the central axis. The stirring vessel 1 comprises a barrel portion 1 b having a uniform inner diameter, a vessel bottom 1 a below the barrel portion 1 b and a vessel top 1 c above the barrel portion 1 b. At the center of the vessel bottom 1 a, an outlet 11 is provided.

The lower end of the stirring shaft 2 is supported by the vessel bottom 1 a via a bearing 3, and the upper end of the stirring shaft 2 is connected, via a coupling 4a, to a drive unit 4 mounted on the vessel top 1 c. The drive unit 4 is connected to an exterior drive source (not shown) and is adapted so that the stirring shaft 2 is rotated by driving the drive source.

Stirring vanes 15 are mounted on the stirring shaft 2. The stirring vanes 15 in this embodiment are ones wherein left and right two bottom paddles 5, 5 located at a lower portion of the stirring shaft 2 and left and right two lattice vanes 6, 6 located above them are integrated.

That is, on a lower portion of the stirring shaft 2, two plate-shaped bottom paddles 5, 5 are mounted which extend in a radial direction of the stirring vessel 1 with the stirring shaft 2 as the base end. As viewed from above, the angle between the two bottom paddles 5, 5 is 180°.

On a portion of the stirring shaft 2 above the position where the bottom paddles 5, 5 are mounted, two lattice vanes 6, 6 are mounted which extend in a radial direction of the stirring vessel 1 with the stirring shaft 2 as the base end and in the same direction as the bottom paddles 5, 5. Each lattice vane 6 is constituted by three plate-shaped arm paddles 7a, 7b, 7c extending in a radial direction and two plate-shaped strips 8a, 8b extending in a central axis direction, which are integrated in a lattice form. The three arm paddles 7a, 7b, 7c are parallel to one another, and two among them constitute the upper side (7a) and the lower side (7c), respectively, and the remaining one (7b) is disposed between the upper and lower sides. The two plate-shaped strips 8a, 8b are parallel to each other, and one of them constitutes an outer edge (8a) in a radial direction of the lattice vane 6, and the remaining one (8b) is disposed between the outer edge and the stirring shaft 2. As viewed from above, the angle between the two lattice vanes 6, 6 is 180°.

In this embodiment, the arm paddle 7c constituting the lower side of the lattice vane 6 is integrated with the bottom paddle 5. That is, the lattice vane 6 and the bottom paddle 5 are integrated.

As such a stirring vane 15 having the lattice vane 6 and the bottom paddle 5 integrated, for example, Max Blend (registered trademark) vane (trade name, manufactured by Sumitomo Heavy Industries Process Equipment Co., Ltd.) may suitably be employed.

The largest value of the width of the stirring vanes 15 (referred to as the entire width of the stirring vanes 15 in this specification) in a radial direction of the stirring vessel 1 is preferably at least 20%, more preferably at least 40%, of the inner diameter of the stirring vessel 1.

Here, the inner diameter of the stirring vessel 1 means the largest diameter of the inner diameter of the stirring vessel 1 and is the inner diameter of the barrel portion 1 b in this embodiment.

In the lattice vane 6, rectangular openings 6a are provided which are defined by the arm paddles 7a, 7b, 7c, the strips 8a, 8b and the rotary shaft 2.

As the stirring vanes 15 are provided with such openings 6a and bottom paddles 5, 5, it is made possible that when the stirring vanes 15 are rotated, a strong downward flow is formed in the vicinity of the rotary shaft 2, and at the vessel bottom 1 a, an emission flow is formed from a lower portion of the rotary shaft 2 towards the inner wall of the stirring vessel 1, and further, an upward flow is formed between the stirring vanes 15 and the inner wall of the stirring vessel 1. Further, the downward flow in the vicinity of the rotary shaft 2 is finely divided and dispersed by the openings 6a.

The size and number of the openings 6a, the size of the bottom paddles 5, 5, etc., may suitably set so that such effects are obtainable to desired levels.

In this embodiment, eight openings 6a are provided in the stirring vanes 15. The total area of the openings 6a may, for example, be preferably from 40 to 90%, more preferably from 60 to 80%, of the total area of the stirring vanes 15 when it is assumed that no openings 6a are provided.

The total area of the bottom paddles 5, 5 is preferably from 5 to 30%, more preferably from 10 to 20%, of the total area of the stirring vanes 15 when it is assumed that no openings 6a are provided.

In this embodiment, a discharge nozzle 10 made of a pipe is provided as a supply means for discharging a monoepoxide (monoepoxide-supply means) to at least two locations below the position of the lower ends of the strips 8a, 8b in the stirring vessel 1. The discharge nozzle 10 has, at its forward end, a ring portion (ring-shaped discharge nozzle) 10a, and at least two discharge openings 10c are open on the pipe wall of the ring portion 10a. The ring portion 10a is communicated with a supply pipe10b passing through the outer wall of the stirring vessel 1 and is communicated, via the supply pipe 10b, with a monoepoxide-supply section (not shown) provided outside of the stirring vessel 1.

The ring portion 10a of the discharge nozzle 10 is disposed to surround the bearing portions 3 between the lower ends 5a of the bottom paddles 5 and the vessel bottom 1 a. The ring portion 10a is fixed to a support member (not shown) provided on the vessel bottom 1 a of the stirring vessel 1, and there is a space between the ring portion 10a and the vessel bottom 1 a so that they will not be in contact with each other. Further, there is a space also between the lower ends 5a of the bottom paddles 5 and the ring portion 10a so that they will not be in contact with each other. That is, the ring portion 10a is provided so that it will not be in contact with both the bottom paddles 5 and the vessel bottom 1 a.

The distance between the ring portion 10a of the discharge nozzle 10 and the vessel bottom 1 a in a central axis direction of the stirring vessel 1 is preferably at most 5%, more preferably at most 2%, to the entire height in a central axis direction of the stirring vessel 1. As this value becomes smaller, the dispersion effect of the monoepoxide tends to be more readily obtainable.

The entire height of the stirring vessel 1 is meant for the height of the longest portion in length from the vessel bottom to the vessel top inside of the stirring vessel.

The distance between the ring portion 10a of the discharge nozzle 10 and the lower ends 5a of the bottom paddles 5 is preferably at most 5%, more preferably at most 2%, to the entire height of the stirring vessel 1. As this value becomes smaller, the effect to improve the dispersibility of the monoepoxide by the use of the stirring vanes having the lattice vanes and the bottom paddles tends to be more readily obtainable.

The distance (hereinafter sometimes referred to also as clearance C) between the lower ends 5a of the bottom paddles 5 and the vessel bottom 1 a is set to be at least a distance whereby a space is secured between the ring portion 10a of the discharge nozzle 10 and the vessel bottom 1 a, and a space is secured between the ring portion 10a and the lower ends 5a of the bottom paddles 5. The upper limit of the clearance C is preferably at most 30%, more preferably at most 15%, of the inner diameter of the stirring vessel 1. When the upper limit is at most the above value, the effect to improve the dispersibility of the monoepoxide by the use of the stirring vanes having the lattice vanes and the bottom paddles tends to be more readily obtainable.

The inner diameter of the pipe constituting the ring portion 10a of the discharge nozzle 10 is not particularly limited, but if it is too large, the stirring efficiency of the stirring vanes tends to be low, and if it is too small, the supply rate of the monoepoxide is limited, and therefore, it is preferably set to avoid such drawbacks. For example, it is preferably from 0.05 to 5%, more preferably from 0.1 to 2%, to the entire height in the central axis direction of the stirring vessel 1.

When the ring portion 10a is viewed from above, the outer diameter of the ring is preferably from 20 to 95%, more preferably from 30 to 90%, of the entire width of the stirring vanes 15 in a radial direction of the stirring vessel 1. If it is less than 20%, the multi-point feeding effect tends to be low, and if it exceeds 95%, the dispersion effect of the monoepoxide tends to be low.

The shape of the discharge openings 10c is not particularly limited, but is preferably circular. The size of the discharge openings 10c is not particularly limited, but if it is too large, the introduction pressure of the monoepoxide tends to be unstable, and if it is too small, the supply rate of the monoepoxide tends to be deficient, and therefore, it is preferably set to avoid such drawbacks. For example, it is preferably from 0.5 to 20 mm, more preferably from 1 to 10 mm.

The number of discharge openings 10c may be at least 2. It is preferably at least 3, more preferably at least 4, in that the monoepoxide may be supplied with better dispersibility, and the effect to prevent the viscosity from becoming high at the time of preparation of the polyether may be large. The upper limit is not particularly limited, but from such a viewpoint that the monoepoxide can be introduced uniformly from each discharge opening, it is preferably at most 20, more preferably at most 15.

In the Fig., reference symbol 9 represents a baffle plate, which is provided on the inner surface of the side wall of the stirring vessel 1. The length direction of a baffle plate 9 is in a central axis direction, and in this embodiment, it is continuous from the lower portion to the upper portion of the side wall of the stirring vessel 1. In a circumferential direction of the stirring vessel 1, a plurality of baffle plates 9 are disposed at equal distances.

Baffle plates 9 are not essential, but by providing them, it is possible to further improve the effect for improvement of the dispersibility of the monoepoxide by the use of the stirring vanes having the lattice vanes and the bottom paddles. The shape, size, disposition, etc. of the baffle plates 9 may suitably be set so that such an effect is obtainable to a desired level.

For example, the length of the baffle plates 9 in a central axis direction of the stirring vessel 1 is preferably from 50 to 100%, more preferably from 60 to 90%, of the entire length in a central axis direction of the stirring vanes 15. The width of the baffle plates 9 in a radial direction of the stirring vessel 1 is preferably from 0.5 to 5%, more preferably from 1 to 3%, of the inner diameter of the stirring vessel 1. The number of the baffle plates 9 in a circumferential direction of the stirring vessel 1 is preferably 2, 4 or 6, more preferably 4 or 6.

In the Fig., reference symbol 12 represents an inlet which is provided at the vessel top 1 c of the stirring vessel 1 and which is designed to be openable and closable.

In the polymerization step, if the stirring speed (rotational speed) of the stirring vanes 15 at the time of stirring while discharging the monoepoxide into the stirring vessel 1, is too low, good dispersibility of the monoepoxide tends to be hardly obtainable, and if it is too high, it tends to be difficult to adapt installations to obtain the required power. Therefore, the stirring speed is set to be within a range not to bring about such drawbacks. For example, when the viscosity of the liquid in the vessel is 500 mPa·s, the stirring speed (rotational speed) is set preferably so that the stirring power (unit power) becomes to be from 0.5 to 500 Kw/m$^3$, more preferably so that it becomes to be from 1 to 300 Kw/m$^3$.

In this embodiment, a case has been described wherein the drive unit 4 for driving the stirring shaft 2 from outside of the vessel is provided on the vessel top side, but the drive unit 4 may be provided on the vessel bottom side.

Further, in this embodiment, as the monoepoxide-supply means, a discharge nozzle 10 provided with the ring portion 10a is employed, but it may have any construction so long as the effect to improve dispersibility of the monoepoxide is obtainable by discharging the monoepoxide to at least two locations below the position of the lower ends of the strips 8a, 8b in the stirring vessel 1. For example, a flow path for the monoepoxide may be provided in the stirring vanes 15 and at the same time, discharge openings may be formed on the outer surface of the bottom paddles 5, 5. As in this embodiment, it is more preferred to have such a construction that the monoepoxide is discharged to below the lower ends of the stirring vanes 15.

The forward end portion of the discharge nozzle 10 may be a straight form or a ring form, but a ring form is preferred from such a viewpoint that many discharge openings 10c may thereby be provided. Especially when at least three discharge openings are to be provided, a ring form is preferred.

The shape of the stirring vanes 15 may be changed within the range of the present invention. In this embodiment, the bottom paddles 5, 5 and the lattice vanes 6, 6 are integrated, but they may be separated. And, in a case where they are separated, when the stirring vanes 15 are viewed from above, the bottom paddles 5, 5 and the lattice vanes 6, 6 may be overlapped or may be crossed.

<Polyether preparation method>

The polyether preparation method of the present invention has a polymerization step of subjecting a monoepoxide having at least 2 carbon atoms to ring-opening addition polymerization to an initiator having at least one active hydrogen-containing functional group in the presence of a catalyst in the stirring vessel.

[Polyether]

The polyether in the present invention means a linear polymer compound having ether bonds in its main chain, obtainable by ring-opening addition polymerization of a monoepoxide having at least two carbon atoms to an initiator having at least one active hydrogen-containing functional group. The monoepoxide is ring-opened and added to the active hydrogen of the active hydrogen-containing functional group to form a hydroxy group anew, and then, to this hydroxy group, the monoepoxide is further ring-opened and added to form an ether bond and at the same time to form a new hydroxy group. Such a ring-opening addition reaction of the monoepoxide is repeated to form a linear polymer compound having ether bonds in its main chain. Accordingly, at least one of the terminal groups of the polyether of the present invention is a hydroxy group.

[Initiator]

Active hydrogen means an active hydrogen atom to which the monoepoxide is reactive, such as a hydrogen atom of a hydroxy group, or a hydrogen atom of an amino group. The active hydrogen-containing functional group may be a hydroxy group, a carboxy group, a mercapto group or an amino group (primary or secondary).
The initiator may be any compound so long as it has at least one active hydrogen-containing functional group, and a compound known as an initiator for a polyether may suitably be used.
As the initiator, it is preferred to use a polyether monol or polyether polyol having a number average molecular weight (Mn) of at least 300 per hydroxy group in view of the DMC catalyst activity during the preparation of the polyether. Such a polyether monol or polyether polyol may contain a chemical bond other than the ether bonds, selected from e.g. an ester bond and a carbonate bond, by an optional selection.
The polyether monol or polyether polyol as the initiator is particularly preferably a polyether monol or polyether polyol obtainable by ring-opening addition polymerization of propylene oxide and/or ethylene oxide to a low molecular weight initiator. The low molecular weight initiator is preferably a monol such as methanol, ethanol, propanol, butanol or hexanol, water, or a polyol such as ethylene glycol, propylene glycol, diethylene glycol, dipropylene glycol, trimethylol propane, glycerine, pentaerythritol, sorbitol or sucrose.
As the initiator, the average number of hydroxy groups per molecule is preferably from 1 to 8, more preferably from 1 to 4. The hydroxy value is preferably from 10 to 600 mgKOH/g, more preferably from 10 to 400 mgKOH/g, particularly preferably from 10 to 240 mgKOH/g.
The hydroxy value (mgKOH/g) in the present invention is a value measured in accordance with JIS K-1557.
In the polymerization step, one of the initiators may be used alone, or two or more of them may be used in combination.

[Monoepoxide]

The monoepoxide is a compound having one epoxy ring. A monoepoxide known in the preparation of a polyether may suitably be used. As specific examples, an alkylene oxide, glycidyl ether, glycidyl ester, etc. may be mentioned. An alkylene oxide is preferred from such a viewpoint that the polyether may thereby be made to have a high molecular weight.
The alkylene oxide having at least two carbon atoms may, for example, be ethylene oxide, propylene oxide, 1,2-butylene oxide, 2,3-butylene oxide or styrene oxide, and ethylene oxide or propylene oxide is preferred.
In the polymerization step, one of the monoepoxides may be used alone, or two or more of them may be used in combination. In a case where two or more monoepoxides are subjected to ring-opening addition polymerization to the initiator, a mixture of two or more monoepoxides may be subjected to ring-opening addition polymerization to let random polymer chains be formed, or two or more monoepoxides may be separately sequentially subjected to ring-opening addition polymerization to let block polymer chains be formed. Otherwise, formation of random polymer chains and formation of block polymer chains may be combined. As a raw material for a modified silicone polymer, it is preferred to use propylene oxide alone.

[Catalyst]

As the catalyst to be used in the polymerization step, a catalyst known in ring-opening addition polymerization of a monoepoxide may suitably be used. For example, a double metal cyanide complex catalyst, a Lewis acid catalyst or an alkali metal catalyst may be mentioned.

Especially when the catalyst is a double metal cyanide complex catalyst, the reaction rate tends to be readily increased, whereby in the preparation method of the present invention, the effect to improve the dispersibility of the monoepoxide and thereby to prevent an increase of the viscosity during the preparation is large. That is, when the catalyst is a double metal cyanide complex catalyst, it is particularly preferred to use the preparation method of the present invention.

The double metal cyanide complex catalyst (hereinafter sometimes referred to as DMC catalyst) is preferably a DMC catalyst having an organic ligand coordinated to zinc hexacyano cobaltate.

The organic ligand in the DMC catalyst may, for example, be an alcohol, an ether, a ketone, an ester, an amine or an amide. As a preferred organic ligand, tert-butyl alcohol, n-butyl alcohol, iso-butyl alcohol, tert-pentyl alcohol, iso-pentyl alcohol, N,N-dimethylacetamide, ethylene glycol mono-tert-butyl ether, ethylene glycol dimethyl ether (also called as glyme), diethylene glycol dimethyl ether (also called diglyme), triethylene glycol dimethyl ether (also called triglyme), iso-propyl alcohol, or dioxane, may be mentioned. The dioxane may be 1,4-dioxane or 1,3-dioxane, but 1,4-dioxane is preferred. One of the organic ligands may be used alone, or two or more of them may be used in combination.

Among them, it is preferred to have tert-butyl alcohol as the organic ligand. Accordingly, it is preferred to use a DMC catalyst having tert-butyl alcohol as at least a part of the organic ligand. The DMC catalyst having such an organic ligand has a high activity, whereby a polyether having a low total degree of unsaturation is readily obtainable. Further, when a DMC catalyst having a high activity is used, it is possible to reduce the amount to be used, such being desirable also with a view to reducing the amount of the remaining catalyst.

In the polymerization step, one of the catalysts may be used alone, or two or more of them may be used in combination. It is more preferred to use one catalyst alone.

[Polymerization step]

The polymerization step is a step wherein using the stirring vessel 1 having the construction as described above, the initiator and the catalyst are filled to the stirring vessel 1, and the monoepoxide is supplied to the stirring vessel 1 to carry out the ring-opening addition polymerization.

It is preferred to supply the monoepoxide into a liquid of the initiator in the initial stage of the polymerization. By supplying the monoepoxide into the initiator liquid, mixing of the initiator and the monoepoxide is facilitated in the initial stage of the polymerization, so that they may be swiftly reacted. Further, as the amount of the monoepoxide reacted increases, the amount of the liquid in the stirring vessel increases, whereby even after the initial stage of the polymerization step, it is possible to continuously supply the monoepoxide into the liquid.

Specifically, in such a state that the catalyst and the entire amount of the initiator are supplied and filled into the stirring vessel 1, discharge openings 10c to discharge the monoepoxide are opened in the liquid of the initiator. Depending upon the amount of the initiator, the height of the liquid surface of the initiator in the stirring vessel 1 changes, and therefore, the open positions of discharge openings 10c in the stirring vessel 1 are preferably set to be below the position of the lower ends of strips and at a position relatively lower among them, in consideration of the change in the height of the initiator liquid surface. As mentioned above, it is particularly preferred to provide the openings between the bottom paddles 5 and the bottom of the stirring vessel 1.

Further, in the polymerization step, it is preferred that the liquid in the stirring vessel is stirred prior to the supply of the monoepoxide. Therefore, the amount of liquid prior to the supply of the monoepoxide in the stirring vessel is adjusted to be at a level where the liquid can be stirred. Specifically, it is preferred that at least the lower ends of the bottom paddles 5 are located in the liquid of the initiator in such a state that the entire amount of the initiator and the DMC catalyst are filled in the stirring vessel 1.

Now, one embodiment of a case where a DMC catalyst is used as the catalyst, will be described.

In this embodiment, using a stirring vessel 1 having a temperature-adjusting function and a pressure-resistant structure, firstly the entire amount of the initiator and the DMC catalyst are supplied into the stirring vessel 1. Preferably after flushing the interior of the stirring vessel 1 with an inert gas such as nitrogen to remove oxygen, the system is heated with stirring to a prescribed reaction temperature.

Then, a part of the monoepoxide is supplied and reacted to activate the DMC catalyst. When the DMC catalyst is activated, heat is generated, and a pressure decrease results, whereby it can be confirmed that the activation has occurred. The amount of the monoepoxide supplied in this step is preferably from 3 to 20 mass%, more preferably from 5 to 15 mass%, to the initiator.

Then, the remaining monoepoxide is gradually supplied and reacted with stirring while maintaining the reaction temperature at a prescribed level. The remaining monoepoxide may be supplied continuously or may be supplied intermittently. If the supply rate of the monoepoxide into the stirring vessel 1 at that time is too high, the monoepoxide tends to be hardly uniformly dispersed, and if it is too low, such tends to lead to a loss of the preparation time, and therefore, the supply rate is preferably set not to bring about such drawbacks. The amount of the monoepoxide to be supplied per hour is preferably from 5 to 30 mass%, more preferably from 10 to 25 mass%, to the amount (100 mass%) of the polyether to be prepared.

After completion of the supply of the monoepoxide, ageing is carried out by maintaining the reaction temperature at the same level with stirring, to obtain a polyether.

The reaction temperature is preferably within a range of from 90 to 150°C, more preferably from 100 to 140°C.

In the polymerization step, a polymerization solvent may be used as the case requires. For example, a solvent as disclosed in Japanese Patent No. 2,946,580 may be used. Specifically, hexane, cyclohexane, benzene or ethyl methyl ketone may be mentioned.

It is possible to adjust the molecular weight of the obtainable polyether by the amount of the monoepoxide to be subjected to ring-opening addition polymerization to the initiator.

After the polymerization step, purification such as removal of the solvent or removal of the catalyst may be carried out as the case requires. Further, an additive e.g. a stabilizer such as an antioxidant may be added.

In the present invention, the amount of the polyether to be prepared, means the total mass of the initiator and the monoepoxide added to the initiator, and the total mass of the initiator and the monoepoxide to be supplied to the stirring vessel is deemed to be the amount of the polyether to be prepared.

In the present invention, the molecular weight of the polyether to be prepared in the polymerization step is not particularly limited. However, the preparation method of the present invention is particularly suitable for preparation of a polyether having a high molecular weight, and it is possible to prepare a polyether having a low viscosity while it has a high molecular weight.

The viscosity of the reaction liquid during the preparation of the polyether increases as the molecular weight of the synthesized polyether increases. Especially when the number average molecular weight of the polyether becomes to be at least 10,000, the increase of the viscosity tends to remarkably proceed, whereby the preparation tends to be difficult, or even if the preparation is possible, the viscosity tends to be so high that the polymer is likely to be not suitable for use. Therefore, as the molecular weight of the polymer becomes high, the demand for a low viscosity becomes high, and it becomes more effective to employ the present invention.

The preparation method of the present invention is suitable for the preparation of a polyether having a number average molecular weight of at least 10,000, more preferably at least 20,000, particularly preferably at least 30,000. The upper limit in the number average molecular weight is not particularly limited, and it is possible to prepare a polyether having a number average molecular weight of up to 80,000, realistically preferably at most 50,000.

Further, the number average molecular weight of the polyether to be prepared, is preferably at least 1.5 times, more preferably at least 2 times, of the number average molecular weight of the initiator used for its preparation. It is possible to prepare a polyether having a number average molecular weight close to 100 times of the number average molecular weight of the initiator, but since the capacity of the stirring vessel is limited by itself, usually the number average molecular weight of the polyether is preferably at most 30 times, more preferably at most 20 times, of the number averge molecular weight of the initiator. In a case where a polyether having a higher molecular weight is to be prepared from an initiator having a relatively low number average molecular weight, it is possible to conduct the preparation by repeating the preparation method of the present invention. That is, a polyether is prepared from the initiator having a relatively low number average molecular weight, and then, using the obtained polyether as an initiator, a polyether having a higher molecular weight can be prepared.

By using the preparation method of the present invention, it is possible to suppress the viscosity of the polyether to be low. The reason is considered to be such that in the polymerization step, good stirring efficiency is obtainable by using the stirring vessel having the above described construction and at the same time, dispersibility of the monoepoxide in the stirring vessel is well improved by supplying the monoepoxide by the method of discharging the monoepoxide from at least two locations in the stirring vessel, and as a result, the molecular weight distribution becomes small, and even if the number average molecular weight is the same, the viscosity becomes low.

Accordingly, it is possible to obtain a polyether having the viscosity lowered with the same number average molecular weight. Thus, a polyether having a high molecular weight which used to be not suitable for use because the viscosity used to be high, can be obtained with a viscosity suitable for use.

The polyether obtained by the preparation method of the present invention is useful for various known applications. For example, it can be suitably used for the preparation of a modified silicone polymer which is useful as a sealant.

<Modified silicone polymer>

The modified silicone polymer of the present invention is one having a structure such that a hydrolysable silyl group represented by the following formula (1) is introduced via a linking group to a terminal of the polyether.

$$-SiX_aR^1_{3-a} \qquad (1)$$

In the formula (1), $R^1$ is a substituted or unsubstituted $C_{1-20}$ monovalent organic group, X is a hydroxy group or a hydrolysable group, and a is 1, 2 or 3, provided that when a plurality of $R^1$ are present, they may be the same or different, and when a plurality of X are present, they may be the same or different.

The hydrolysable group as X in the formula (1) may, for example, be a halogen atom, an alkoxy group, an acyloxy group, an amido group, an amino group, an aminoxy group, a ketoxymate group or a hydride group.

The carbon number of a hydrolysable group having carbon atoms among them, is preferably at most 6, more preferably at most 4. X is preferably a lower alkoxy group having at most 4 carbon atoms, and a methoxy group, an ethoxy group, a propoxy group or a propenyloxy group is particularly preferred.

In the formula (1), $R^1$ is preferably an alkyl group having at most 8 carbon atoms, a phenyl group or a fluoroalkyl group. Particularly preferred are a methyl group, an ethyl group, a propyl group, a butyl group, a hexyl group, a cyclohexyl group, a phenyl group, etc.

<Modified silicone polymer preparation method>

The modified silicone polymer preparation method of the present invention comprises a step of preparing a polyether by the preparation method of the present invention, and a step of introducing a hydrolysable silyl group to a molecular terminal of the polyether.

As the method of introducing a hydrolysable silyl group to a molecular terminal of the polyether, a known method may be employed. For example, it is possible to employ one of the following methods (i) to (iv).

[Method (i)]

In the polymerization step, a polyether having a hydroxy group at a terminal is prepared, an olefin group is introduced to the terminal, and then, a hydrosilyl group represented by the following formula (2) is reacted, whereby a hydrolysable silyl group can be introduced.

$$HSiX_aR^1{}_{3-a} \qquad (2)$$

In the formula (2), $R^1$, X and a are as defined above.

As a method of introducing an olefin group to the polyether, it is possible to employ, for example, a method wherein a compound having an olefin group and a functional group reactive with a hydroxy group, is reacted to the hydroxy group of the polyether.

[Method (ii)]

In the polymerization step, at the time of ring-opening addition polymerization of the monoepoxide to the initiator, an olefin group-containing epoxy compound such as allyl glycidyl ether is subjected to ring-opening addition polymerization to prepare an allyl group-modified polyether having an olefin group (e.g. an allyl group) introduced to a terminal of the polyether, and the hydrosilyl compound represented by the above formula (2) is reacted thereto, whereby a hydrolysable silyl group can be introduced.

[Method (iii)]

In the polymerization step, a polyether having a hydroxy group at a terminal is prepared, this polyether is reacted with a compound having a polyisocyanate group and the hydrolysable silyl group represented by the above formula (1), whereby a hydrolysable silyl group can be introduced.

[Method (iv)]

By the above method (i) or (ii), a polyether having an olefin group introduced to its terminal is obtained, and the olefin group is reacted with a mercapto group (-SH) of a silicon compound represented by the following formula (3), whereby a hydrolysable silyl group can be introduced.

$$R^1{}_{3-a}\text{-}SiX_a\text{-}R^2SH \qquad (3)$$

In the formula (3), $R^1$, X and a are as defined above, and $R^2$ is a bivalent organic group.

<Prepolymer preparation method and modified silicone polymer preparation method employing it>

The prepolymer preparation method of the present invention comprises a step of preparing a polyether by the preparation method of the present invention and a step of reacting the polyether and a polyisocyanate compound to obtain a prepolymer having an isocyanate group at its terminal.

The modified silicone polymer preparation method of the present invention employing the prepolymer comprises a step of preparing a prepolymer by the preparation method of the present invention and a step of introducing a hydrolysable silyl group to a molecular terminal of the prepolymer.

The polyisocyanate compound may, for example, be an aromatic polyisocyanate such as naphthalene-1,5-diisocyanate, polyphenylene polymethylene polyisocyanate, 4,4'-diphenylmethane diisocyanate, 2,4-tolylene diisocyanate or 2,6-tolylene diisocyanate; an aralkyl polyisocyanate such as xylylene diisocyanate or tetramethylxylylene diisocyanate; an aliphatic polyisocyanate such as hexamethylene diisocyanate, 2,2,4-trimethyl-hexamethylene diisocyanate or 2,4,4-trimethyl-hexamethylene diisocyanate; an alicyclic polyisocyanate such as isophorone diisocyanate or 4,4'-methylene bis(cyclohexyl isocyanate); or a urethane modified product, burette modified product, allophanate modified product, carbodiimide modified product or isocynurate modified product obtainable from the above polyisocyanate compound.

Among them, one having two isocyanate groups is preferred, and 2,4-tolylene diisocyanate, 2,6-tolylene diisocyanate, hexamethylene diisocyanate or isophorone diisocyanate is preferred.

By reacting the polyether and the polyisocyanate compound in an isocyanate group-excessive proportion, a prepolymer having an isocyanate group at its terminal is obtainable. This step may be carried out by means of a known method.

As the method of introducing a hydrolysable silyl group to a molecular terminal of the prepolymer having an isocyanate group at its terminal, a known method may be used. For example, the following method (v) may be used.

[Method (v)]

To the terminal isocyanate group of the prepolymer, a W group of a silicon compound represented by the following formula (4) is reacted, whereby a hydrolysable silyl group can be introduced.

$$R^1_{3-a}\text{-}SiX_aR^2W \qquad (4)$$

In the formula (4), $R^1$, X and a are as defined above, $R^2$ is a bivalent organic group, and W is an active hydrogen-containing group selected from a hydroxy group, a carboxy group, a mercapto group and an amino group (primary or secondary).

By preparing a prepolymer or modified silicone polymer by using the polyether obtained by the preparation method of the present invention, it is possible to obtain a prepolymer or modified silicone polymer having the viscosity lowered with the same number average molecular weight as ever. It is thereby possible that a modified silicone polymer having a high molecular weight which used to be not suitable for use because the viscosity used to be too high, is obtainable with a viscosity suitable for use.

When the viscosity of a modified silicone polymer is lowered, the coating properties of the polymer will be improved. Further, when the molecular weight of a modified silicone polymer is high, the cured product will be excellent in its mechanical properties such as the strength and elongation.

Such a modified silicone polymer is suitable particularly for a sealant.

EXAMPLES

Now, the present invention will be described in further detail with reference to Examples, but it should be understood that the present invention is by no means limited to these Examples.

<Measurement methods and evaluation methods>

(1) Viscosity

The viscosity at 25°C was measured in accordance with JIS K-1557 by means of an E-type viscometer VISCONIC EHD Model (manufactured by Tokimec Inc.) and using No. 1 rotor.

(2) Total degree of unsaturation (USV)

Measured by a mercury acetate method in accordance with JIS K-1557.

(3) Number average molecular weight (Mn) and mass average molecular weight (Mw)

The number average molecular weight (Mn), the mass average molecular weight (Mw) and the molecular weight distribution (Mw/Mn) of a polyether are molecular weights calculated as polystyrene, as obtained by measurement by gel permeation chromatography (GPC) under the following conditions by means of a calibration curve prepared by using a standard polystyrene sample with a known molecular weight. [GPC measurement conditions] Type of machine used: HLC-8220GPC (manufactured by Tosoh Corporation), data processing apparatus: SC-8020 (manufactured by Tosoh Corporation), column employed: TSG gel G2500H (manufactured by Tosoh Corporation), column temperature: 40°C, detector: RI, solvent: tetrahydrofuran, flow rate: 0.6 ml/min., sample concentration: 0.25%, injected amount: 10 $\mu$l, standard sample for preparation of a calibration curve: polystyrene ([EasiCal] PS-2 [Polystyrene Standards], manufactured by Polymer Laboratories Ltd.)

(4) Real number of functional groups

The real number f of functional groups in a polymer was obtained by the following formula.

$$f = (1000\ fn/Mn)/[\{(1000/Mn) - (USV/fn)\} + USV]$$

wherein fn is the number of hydroxy groups in the initiator, Mn is the number average molecular weight, and USV is the total degree of unsaturation.
As formation of a by-product monol becomes small, the difference between the number of hydroxy groups and the real number of functional groups in the initiator becomes small.

(5) Presence or absence of gelled substance in polyether

The obtained polyether and the autoclave and stirring vanes after the preparation were visually observed, and the presence or absence of gelled substance was confirmed.

[EXAMPLE 1]

In this Example, a polyether was prepared by using a stirring vessel 1 having the construction as shown in Fig. 1. As the stirring vessel 1, a SUS autoclave (inner diameter: 208 mm, height: 350 mm) equipped with an external heating medium jacket and an internal coil for cooling, was used.
As the stirring vanes 15 having lattice vanes 6 and bottom paddles 5 integrated, as shown in Fig. 1, Max Blend (registered trademark) vanes (trade name, manufactured by Sumitomo Heavy Industries Process Equipment Co., Ltd.) were used. In a central axis direction of the stirring vessel 1, the entire length of each stirring vane 15 is 310 mm, the length of each of the arm paddle 7a constituting the upper side of the lattice vane 6 and the arm paddle 7b therebelow is 15 mm, and the total length of the arm paddle 7c constituting the lower side and the bottom paddle 5 is 55 mm. The distance from the lower end of the arm paddle 7a to the upper end of the arm paddle 7b is 110 mm, and the distance from the lower end of the arm paddle 7b to the upper end of the arm paddle 7c is 115 mm.
In a radial direction of the stirring vessel 1, the entire width of the stirring vanes 15 is 116 mm, the width of each of the two strips 8a, 8b is 9 mm, and the distance from the central axis to the inside end of the inside strip 8b is 29 mm.
The stirring vanes 15 are mounted on the stirring shaft so that the distance (clearance C) between the lower end 5a of the bottom paddle 5 and the vessel bottom 1 a becomes to be 20 mm.
In a circumferential direction of the stirring vessel 1, four baffle plates 9 are provided at equal intervals. The size of each baffle plate is such that the length in a central axis direction of the stirring vessel 1 is 10 mm, and the width in a radial direction is 260 mm.
The discharge nozzle 10 is constituted by a pipe made of SUS and having an inner diameter of 4 mm, and its forward end constitutes a ring portion 10a. The outer diameter of the ring portion 10a is 110 mm. When the ring portion 10a is viewed from above, on the pipe wall of its outer edge, four circular discharge openings 10c each having an inner diameter of 2 mm are provided at equal intervals in a circumferential direction of the ring portion 10a.
The discharge nozzle 10 is provided so that in a central axis direction of the stirring vessel 1, the distance between the ring portion 10a of the discharge nozzle 10 and the vessel bottom 1 a becomes 7 mm, and the distance between the ring portion 10a of the discharge nozzle 10 and the lower end 5a of the bottom paddle 5 becomes 7 mm.

As the initiator, a polypropylene glycol having a hydroxy value of 112.2 mgKOH/g (molecular weight obtained from the hydroxy value: about 1,000, molecular weight per hydroxy group: about 500) was used, and as the catalyst, a DMC catalyst having tert-butyl alcohol (hereinafter sometimes referred to as TBA) as the organic ligand was used. As the monoepoxide, propylene oxide (hereinafter sometimes referred to as PO) was used. Each of these materials is liquid at the temperature used in this Example.

Into the stirring vessel 1, 900 g of the initiator and 0.45 g of the catalyst were charged, and flushing with nitrogen was carried out to remove oxygen in the system, whereupon the stirring vanes 15 were driven to initiate stirring and mixing. The stirring speed (rotational speed) of the stirring vanes 15 was set so that the stirring power (unit power) would become 1.98 Kw/m$^3$ when the viscosity of the liquid in the vessel was 500 mPa·s (the same applies hereinafter). In this Example, it was 300 rpm.

By heating with stirring, the liquid temperature in the vessel was raised to 130°C, and in order to activate the catalyst, firstly 90 g of PO (initial PO) was supplied over 10 minutes via the discharge nozzle 10. After confirming the activation of the catalyst by heat generation and pressure decrease in the vessel, 8,010 g of PO (the remaining PO) was continuously supplied over 4 hours via the discharge nozzle 10 while maintaining the liquid temperature in the vessel at 130°C. The supply rate of PO during such continuous supply for 4 hours was 22.3 mass% per hour to the amount of the polyether prepared.

After completion of the supply of PO, ageing was carried out by maintaining the temperature at 130°C with stirring, to obtain a polyether. The amount of the polyether prepared was 9,000 g.

With respect to the obtained polyether, the number average molecular weight (Mn), the viscosity, the molecular weight distribution (Mw/Mn), the total degree of unsaturation and the real number of functional groups, were measured by the above methods. Further, the presence or absence of a gelled substance in the obtained polyether was evaluated by the above method.

The results are shown in Table 1. In Table 1, the main preparation conditions are also shown (the same applies thereinafter).

[EXAMPLE 2]

In Example 1, the initiator was changed to a polypropylene glycol having a hydroxy value of 11.2 mgKOH/g (molecular weight obtained from the hydroxy value: about 10,000, molecular weight per hydroxy group: about 5,000).

Further, except that the amount of the initiator was changed to 2,000 g, the amount of the catalyst was changed to 0.35 g, the amount of the initial PO was changed to 100 g and the amount of the remaining PO was changed to 4,900 g, the preparation was carried out by the same installation and preparation method as in Example 1. The amount of the polyether prepared was 7,000 g, and the supply rate of PO during the continuous supply for 4 hours was 17.5 mass% per hour to the amount of the polyether prepared. The measured results by the above methods with respect to the obtained polyether are shown in Table 1.

[EXAMPLE 3]

In Example 2, except that the amount of the catalyst was changed to 0.45 g and the amount of the remaining PO was changed to 6,900 g, the preparation was carried out by the same installation and preparation method as in Example 2. The amount of the polyether prepared was 9,000 g, and the supply rate of PO during the continuous supply for 4 hours was 19.2 mass% per hour to the amount of the polyether prepared. The measured results by the above methods with respect to the obtained polyether are shown in Table 1.

[EXAMPLE 4]

In Example 1, the initiator was changed to a polyether polyol (a polyol obtainable by ring-opening addition polymerization of PO to glycerine) having a hydroxy value of 33.66 mgKOH/g (molecular weight obtained from the hydroxy value: about 5,000, molecular weight per hydroxy group: about 1,700).

Further, except that the amount of the initiator was changed to 1,500 g, the amount of the catalyst was changed to 0.45 g, the amount of the initial PO was changed to 150 g and the amount of the remaining PO was changed to 7,350 g, the preparation was carried out by the same installation and preparation method as in Example 1. The amount of the polyether prepared was 9,000 g, and the supply rate of PO during the continuous supply for 4 hours was 20.4 mass% per hour to the amount of the polyether prepared. The measured results by the above methods with respect to the obtained polyether are shown in Table 1.

[EXAMPLE 5]

In Example 4, except that the amount of the initiator was changed to 1,000 g, the amount of the catalyst was changed to 0.3 g, the amount of the initial PO was changed to 100 g and the amount of the remaining PO was changed to 6,900 g, the preparation was carried out by the same installation and preparation method as in Example 4. The amount of the polyether prepared was 8,000 g, and the supply rate of PO during the continuous supply for 4 hours was 19.2 mass% per hour to the amount of the polyether prepared. The measured results by the above methods with respect to the obtained polyether are shown in Table 1.

[EXAMPLE 6]

In Example 1, the initiator was changed to a polyether polyol (a polyol obtainable by ring-opening addition polymerization of PO to pentaerythritol) having a hydroxy value of 44.88 mgKOH/g (molecular weight obtained from the hydroxy value: about 5,000, molecular weight per hydroxy group: about 1,250).

Further, except that the amount of the initiator was changed to 1,000 g, the amount of the catalyst was changed to 0.4 g, the amount of the initial PO was changed to 100 g and the amount of the remaining PO was changed to 6,900 g, the preparation was carried out by the same installation and preparation method as in Example 1. The supply rate of PO during the continuous supply for 4 hours was 21.6 mass% per hour to the amount of the polyether prepared. The measured results by the above methods with respect to the obtained polyether are shown in Table 1.

[EXAMPLE 7]

In Example 6, except that the amount of the initiator was changed to 900 g, the amount of the catalyst was changed to 0.45 g, the amount of the initial PO was changed to 90 g and the amount of the remaining PO was changed to 8,010 g, the preparation was carried out by the same installation and preparation method as in Example 6. The supply rate of PO during the continuous supply for 4 hours was 22.3 mass% per hour to the amount of the polyether prepared. The measured results by the above methods with respect to the obtained polyether are shown in Table 2.

[COMPARATIVE EXAMPLE 1]

In this Example, in the stirring vessel as shown in Fig. 1, as the monoepoxide-supply means, a single pipe (pipe having an inner diameter of 4 mm) having one discharge opening was used instead of the discharge nozzle 10. That is, the ring portion 10a of the discharge nozzle 10 was removed, so that the monoepoxide was supplied from the forward end of the supply pipe 10b (the same applies hereinafter). Otherwise, the preparation was carried out in the same manner as in Example 4. The measured results by the above methods with respect to the obtained polyether are shown in Table 2.

[EXAMPLE 8]

[Preparation of isocyanate group-terminal prepolymer]

Into a 1 L glass reactor equipped with stirring vanes, 400 g of the polyether obtained in Example 1 is introduced. Further, to the reactor, tolylene diisocyanate (an isomer mixture of 2,4-isomer and 2,6-isomer containing 80 mass% of 2,4-isomer; trade name: TDI-80, Nippon Polyurethane Industry Co., Ltd.) and 4,4'-diphenylmethane diisocyanate (trade name: Milionate MT, Nippon Polyurethane Industry Co., Ltd.) are introduced in a molar ratio of 7/3 and in such amounts that the isocyanate group/hydroxy group (molar ratio) to the polyether will be 1.95. After flushing the interior of the reactor with nitrogen, while stirring the content at 100 rpm, the reactor is heated to 90°C and held as it is at 90°C. During the reaction, a part of the content is sampled every predetermined time, the isocyanate group content $z_1$ (mass%) is measured, and the isocyanate reaction rate $z$ (%) to the theoretical isocyanate group content $z_0$ (mass%) is obtained. By confirming that the isocyanate group content $z_1$ (mass%) has become at most the theoretical isocyanate group content $z_0$ (0.84 mass%), the reaction is terminated to obtain an isocyanate group-terminal prepolymer. The viscosity of the obtained isocyanate group-terminal prepolymer is 44,000 mPa·s.

[EXAMPLE 9]

[Preparation of modified silicone polymer (a)]

Into a SUS autoclave (internal capacity: 5 L (litters)), 3,000 g of the polyether obtained in Example 2 is introduced and subjected to reduced pressure and dehydration while maintaining the internal temperature at 110°C. Then, the internal atmosphere of the reactor is flushed with nitrogen gas, and while maintaining the internal temperature at 50°C, Nahsemu zinc (manufactured by Nippon Kagaku Sangyo Co., Ltd.) is added as a urethane-forming catalyst in an amount of 50 ppm to the polyether, followed by stirring, and then, 1-isocyanate methyl methyldimethoxy silane (purity: 95%) is introduced so that the ratio (NCO/OH) of the total number of isocynate groups to the total number of hydroxy groups will be 0.97. Then, the internal temperature is maintained at 80°C for 8 hours, whereby the polyether and the 1-isocyanate methyl methyldimethoxy silane are subjected to a urethane-forming reaction, whereupon by FT-IR (Fourier transform infrared spectroscopy), it is confirmed that a peak of the isocyanate has disappeared. Then, the system is cooled to room temperature to obtain a modified silicone polymer (a) having a methyldimethoxy silyl group as a hydrolysable group at its terminal. The viscosity of the obtained modified silicone polymer is 60,000 mPa·s.

[Preparation of modified silicone polymer (b)]

Into a SUS autoclave (internal capacity: 5 L (litters)), 3,000 g of the polyether obtained in Example 2 is introduced and subjected to reduced pressure and dehydration while maintaining the internal temperature at 110°C. Then, the internal atmosphere of the reactor is flushed with nitrogen gas, and while maintaining the internal temperature at 50°C, Nahsemu zinc (manufactured by Nippon Kagaku Sangyo Co., Ltd.) is added as a urethane-forming catalyst in an amount of 50 ppm to the polyether, followed by stirring, and then, 3-isocyanate propyl trimethoxy silane (purity: 98%) is introduced so that the ratio (NCO/OH) of the total number of isocynate groups to the total number of hydroxy groups will be 0.97. Then, the internal temperature is maintained at 80°C for 8 hours, whereby the polyether and the 3-isocyanate propyl trimethoxy silane are subjected to a urethane-forming reaction, whereupon by FT-IR (Fourier transform infrared spectroscopy), it is confirmed that a peak of the isocyanate has disappeared. Then, the system is cooled to room temperature to obtain a modified silicone polymer (b) having a methyldimethoxy silyl group as a hydrolysable group at its terminal. The viscosity of the obtained modified silicone polymer (b) is 58,000 mPa·s.

[EXAMPLE 10]

[Preparation of modified silicone polymer (c)]

Into a SUS autoclave (internal capacity: 5 L (litters)), 3,000 g of the polyether obtained in Example 2 is introduced and subjected to reduced pressure and dehydration while maintaining the internal temperature at 110°C. Then, the internal temperature is adjusted to be at 50°C, a methanol solution containing sodium methoxide in an amount of 1.05 times by mol to the amount of hydroxyl groups in the polyether, is added. The temperature is adjusted to 130°C, and methanol is removed under reduced pressure, to carry out an alcholate-forming reaction of the polyether. Then, the liquid temperature is adjusted to 80°C, and allyl chloride is added and reacted in an excess amount to the amount of hydroxy groups of the polyether, followed by removal of unreacted allyl chloride and purification to obtain a polymer having an ally group at its molecular terminal. Then, in the presence of chloroplatinic acid hexahydrate, dimethoxymethyl silane is added in an amount of 0.6 time by mol to the amount of terminal allyl groups in the obtained polymer, followed by a reaction at 70°C for 5 hours, thereby to obtain a modified silicone polymer (c) having a methyldimethoxy silyl group as a hydrolysable group at its terminal. The viscosity of the obtained modified silicone polymer (c) is 50,000 mPa·s.

[EXAMPLE 11]

[Preparation of sealant]

100 Parts by mass of the modified silicone polymer (b) obtained in Example 10, 40 parts by mass of diisononyl phthalate (DINP, trade name: Vinycizer 90, manufactured by Kao Corporation), 75 parts by mass of colloid calcium carbonate (trade name: Hakureika CCR, Shiraishi Kogyo K.K.), 75 parts by mass of heavy calcium carbonate (trade name: Whitone SB, average particle size: 1.78 μm, manufactured by Shiraishi Kogyo K.K.), 5 parts by mass of a fatty acid amide type thixotropy-imparting agent (trade name: Dispalone #6500, Kusumoto Chemicals, Ltd.), 1 part by mass of a benzotriazole type ultraviolet absorber (trade name: Tinuvin 326, manufactured by Ciba Specialty Chemicals Corporation), 1 part by mass of a hindered phenol type antioxidant (trade name: Irganox 1010, manufactured by Ciba Specialty Chemicals Corporation), and 2 parts by mass of a tetravalent organic tin compound (EXCESTAR C201, manufactured by Asahi Glass Company, Limited) are mixed to prepare a one-component system modified silicone type sealant. The one-component system modified silicone type sealant thus obtainable is excellent in workability.

[REFERENCE EXAMPLE 1]

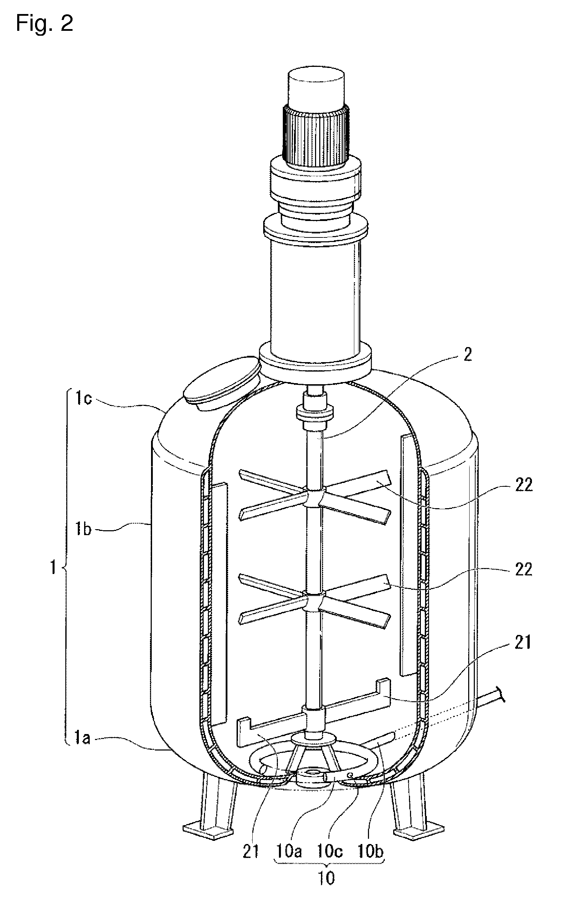

In this Example, in the stirring vessel shown in Fig. 1, the stirring vanes 15 were changed to stirring vanes composed of a combination of anchor vanes 21 and paddle vanes 22 as shown in Fig. 2. Other constituting elements are the same as in Fig. 1, and the same constituting elements are identified by the same reference symbols. Here, reference symbols are omitted except for the main reference symbols.

In this Example, two anchor vanes 21, 21 are mounted on a lower portion of the stirring shaft 2, and above them, two sets of paddle vanes 22 are mounted with a distance from each other, such that in each set, four impeller blades are disposed at equal intervals in the rotational direction.

In a radial direction of the stirring vessel 1, the entire width of the anchor vanes 21, 21 (the total of two vanes) is 116 mm, the entire width of the paddle vanes 22 (the total of two impeller blades) is 116 mm. Each impeller blade of the paddle vanes 22 is a rectangular plate with a short side of 15 mm, which is mounted as inclined at an angle of 45° to a central axis direction.

The distance (clearance C) between the lower end of the anchor vanes 21 and the vessel bottom 1 a is 20 mm.

The preparation was carried out in the same manner as in Example 4 except that the stirring vanes were changed as described above. Here, the stirring speed (rotational speed) of the stirring vanes was set so that the stirring power (unit power) when the viscosity of the liquid in the vessel was 500 mPa·s would be 1.98 Kw/m$^2$, and found to be 400 rpm in this Example.

The measured results by the above methods with respect to the obtained polyether are shown in Table 2. In Table 2, the results in Example 4 are also shown for the purpose of comparison.

[REFERENCE EXAMPLE 2]

In this Example, in the stirring vessel as shown in Fig. 2, as the monoepoxide-supply means, a single pipe (pipe having an inner diameter of 4 mm) having one discharge opening was used instead of the discharge nozzle 10.

Otherwise, the preparation was carried out in the same manner as in Reference Example 1. The measured results by the above methods with respect to the obtained polyether are shown in Table 2.

[REFERENCE EXAMPLE 3]

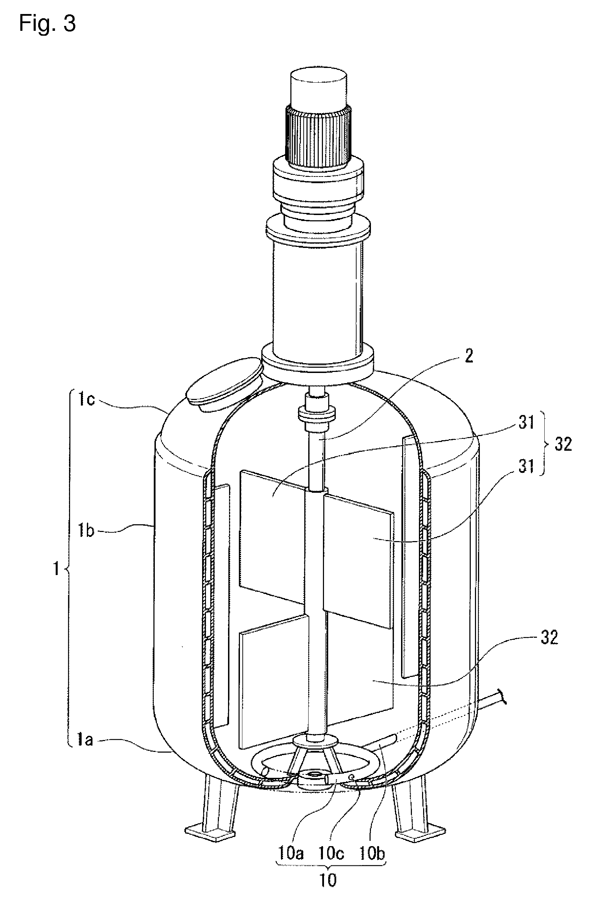

In this Example, in the stirring vessel shown in Fig. 1, the stirring vanes 15 were changed to Full-zone (registered trademark) vanes (manufactured by Kobelco Eco-solutions Co., Ltd.) as shown in Fig. 3. Other constituting elements are the same as in Fig. 1, and the same constituting elements are identified by the same reference symbols. Here, reference symbols are omitted except for the main reference symbols.

In this Example, on the stirring shaft 2, two sets of vanes 32 are mounted continuously in a central axis direction and to cross each other when viewed from above, such that in each set, two impeller blades 31, 31 constitute a rectangular plate as a whole. In a radial direction of the stirring vessel 1, the entire width of the vanes 32 (the total of two impeller blades) is 116 mm. The distance (clearance C) between the lower end of the lower vanes 32 and the vessel bottom 1 a is 20 mm.

The preparation was carried out in the same manner as in Example 4 except that the stirring vanes were changed as described above. Here, the stirring speed (rotational speed) of the stirring vanes was set so that the stirring power (unit power) when the viscosity of the liquid in the vessel was 500 mPa·s would be 1.98 Kw/m$^2$, and found to be 300 rpm in this Example.

The measured results by the above methods with respect to the obtained polyether are shown in Table 2.

[REFERENCE EXAMPLE 4]

In this Example, in the stirring vessel as shown in Fig. 3, as the monoepoxide-supply means, a single pipe (pipe having an inner diameter of 4 mm) having one discharge opening was used instead of the discharge nozzle 10. Otherwise, the preparation was carried out in the same manner as in Reference Example 3. The measured results by the above methods with respect to the obtained polyether are shown in Table 2.

TABLE 1

|  |  | Ex. 1 | Ex. 2 | Ex. 3 | Ex. 4 | Ex. 5 | Ex. 6 | Ex. 7 |
|---|---|---|---|---|---|---|---|---|
| Initiator | Number of hydroxy groups | 2 | 2 | 2 | 3 | 3 | 4 | 4 |

(continued)

|  |  | Ex. 1 | Ex. 2 | Ex. 3 | Ex. 4 | Ex. 5 | Ex. 6 | Ex. 7 |
|---|---|---|---|---|---|---|---|---|
| Polyether | Number average molecular weight (Mn) | 10,000 | 35,000 | 45,000 | 30,000 | 40,000 | 40,000 | 50,000 |
|  | Viscosity [mPa·s] | 4,200 | 45,000 | 110,000 | 33,000 | 99,000 | 50,000 | 90,000 |
|  | Mw/Mn | 1.04 | 1.12 | 1.15 | 1.07 | 1.08 | 1.08 | 1.09 |
|  | Total degree of unsaturation [meq/g] | 0.007 | 0.007 | 0.006 | 0.007 | 0.006 | 0.007 | 0.006 |
|  | Real number of functional groups | 1.93 | 1.79 | 1.73 | 2.63 | 2.47 | 3.30 | 3.17 |
|  | Presence or absence of gelled substance | Absent | Absent | Absent | Absent | Absent | Absent | Absent |
| Preparation conditions | Stirring vessel | Fig. 1 | Fig. 1 | Fig. 1 | Fig. 1 | Fig. 1 | Fig. 1 | Fig. 1 |
|  | Stirring speed [rpm] | 300 | 300 | 300 | 300 | 300 | 300 | 300 |
|  | Unit power [Kw/m³] | 1.98 | 1.98 | 1.98 | 1.98 | 1.98 | 1.98 | 1.98 |
|  | Number of discharge openings | 4 | 4 | 4 | 4 | 4 | 4 | 4 |

TABLE 2

|  |  | Ex. 4 | Comp. Ex. 1 | Ref. Ex. 1 | Ref. Ex. 2 | Ref. Ex. 3 | Ref. Ex. 4 |
|---|---|---|---|---|---|---|---|
| Initiator | Number of hydroxy groups | 3 | 3 | 3 | 3 | 3 | 3 |
| Polyether | Number average molecular weight (Mn) | 30,000 | 30,000 | 30,000 | 30,000 | 30,000 | 30,000 |
|  | Viscosity [mPa·s] | 33,000 | 38,000 | 41,000 | 45,000 | 36,000 | 40,000 |
|  | Mw/Mn | 1.07 | 1.13 | 1.19 | 1.21 | 1.09 | 1.18 |
|  | Total degree of unsaturation [meq/g] | 0.007 | 0.007 | 0.007 | 0.007 | 0.007 | 0.007 |
|  | Real number of functional groups | 2.63 | 2.63 | 2.63 | 2.63 | 2.63 | 2.63 |
|  | Presence or absence of gelled substance | Absent | Present | Present | Present | Present | Present |
| Preparation conditions | Shape of stirring vanes | Fig. 1 | Fig. 1 | Fig. 2 | Fig. 2 | Fig. 3 | Fig. 3 |
|  | Stirring speed [rpm] | 300 | 300 | 400 | 400 | 300 | 300 |
|  | Unit power [Kw/m³] | 1.98 | 1.98 | 1.98 | 1.98 | 1.98 | 1.98 |
|  | Number of discharge openings | 4 | 1 | 4 | 1 | 4 | 1 |

As the results in Tables 1 and 2 show, in Examples 1 to 7 of the present invention, even in the case of a high molecular weight polyether having a number average molecular weight of at least 10,000, it was possible to prevent the viscosity from becoming high during the preparation, and it was possible to carry out the preparation satisfactorily without formation of a gelled substance in the polyether.

As the results in Table 2 show, Example 4 and Comparative Example 1 are examples wherein Max Blend (registered trademark) vanes as shown in Fig. 1 were used. By increasing the number of discharge openings to supply the monoepoxide from 1 (Comparative Example 1) to 4 (Example 4), the viscosity of the polyether was effectively lowered.

Further, no formation of a gelled substance in the polyether was observed.

Reference Examples 1 and 2 are examples wherein paddle vanes as shown in Fig. 2 were used. By increasing the number of discharge openings from 1 (Reference Example 2) to 4 (Reference Example 1), lowering of the viscosity of the polyether was observed, but the degree of lowering of the viscosity was inadequate as compared with Example 4, and formation of a gelled substance was observed in the polyether. The gelled substance is considered to be a by-product having a ultrahigh molecular weight.

Reference Examples 3 and 4 are examples wherein Full-zone (registered trademark) vanes as shown in Fig. 3 were used. By increasing the number of discharge openings from 1 (Reference Example 4) to 4 (Reference Example 3), lowering of the viscosity of the polyether was observed, but the degree of lowering of the viscosity was inadequate as compared with Example 4, and formation of a gelled substance was observed in the polyether.

The entire disclosure of Japanese Patent Application No. 2011-241910 filed on November 4, 2011 including specification, claims, drawings and summary is incorporated herein by reference in its entirety.

REFERENCE SYMBOLS

| | |
|---|---|
| 1: | Stirring vessel |
| 2: | Stirring shaft |
| 5: | Bottom paddle |
| 6: | Lattice vane |
| 7a, 7b, 7c: | Arm paddles |
| 8a, 8b: | Strips |
| 10: | Discharge nozzle (monoepoxide-supply means) |
| 10c: | Discharge opening |
| 15: | Stirring vane |

**Claims**

1.  A polyether preparation method which comprises subjecting a monoepoxide having at least 2 carbon atoms to ring-opening addition polymerization to an initiator having at least one active hydrogen-containing functional group in the presence of a catalyst in a stirring vessel, to obtain a polyether, wherein the stirring vessel is a stirring vessel wherein a stirring shaft rotatable by an external drive source is provided at the center of the stirring vessel; plate-shaped bottom paddles extending in a radial direction of the stirring vessel are mounted on a lower portion of the stirring shaft; lattice vanes each comprising arm paddles extending in a radial direction and strips extending in an axial direction, are mounted on a portion of the stirring shaft above the bottom paddles; and a monoepoxide-supply means for discharging the monoepoxide to at least two locations below the lower ends of the strips in the stirring vessel.

2.  The polyether preparation method according to Claim 1, wherein the polyether has a number average molecular weight of at least 10,000.

3.  The polyether preparation method according to Claim 1 or 2, wherein the catalyst is a double metal cyanide complex catalyst.

4.  The polyether preparation method according to any one of Claims 1 to 3, wherein the monoepoxide-supply means has discharge openings for discharging the monoepoxide, and the discharge openings are provided below the position of the lower ends of the strips of the lattice vanes.

5.  The polyether preparation method according to Claim 4, wherein the discharge openings are present between the bottom paddles and the bottom of the stirring vessel, and the monoepoxide-supply means is provided so as not to be in contact with both of the bottom paddles and the bottom of the stirring vessel.

6.  The polyether preparation method according to any one of Claims 1 to 5, wherein the monoepoxide-supply means is provided with a ring-shaped discharge nozzle.

7.  The polyether preparation method according to any one of Claims 1 to 6, wherein into the stirring vessel, the initiator is filled in such an amount that the liquid surface of the initiator becomes higher than the position of the discharge openings for discharging the monoepoxide, and then, the monoepoxide is supplied into the liquid and subjected to the ring-opening addition polymerization.

8. The polyether preparation method according to any one of Claims 1 to 7, wherein after filling the double metal cyanide complex catalyst and the initiator into the stirring vessel, a part of the monoepoxide is supplied to activate the double metal cyanide complex catalyst, and after activation of the double metal cyanide complex catalyst, the rest of the monoepoxide is supplied and subjected to the ring-opening addition polymerization.

9. The polyether preparation method according to Claim 8, wherein the amount of the monoepoxide supplied to activate the double metal cyanide complex catalyst is from 3 to 20 mass% to the initiator.

10. The polyether preparation method according to Claim 8 or 9, wherein the supply rate of the monoepoxide supplied into the stirring vessel after activation of the double metal cyanide complex catalyst is from 5 to 30 mass%/hr to the amount of the polyether to be prepared.

11. A modified silicone polymer preparation method which comprises a step of preparing a polyether by the preparation method as defined in any one of Claims 1 to 10 and a step of introducing a hydrolysable silyl group to a molecular terminal of the polyether.

12. A prepolymer preparation method which comprises a step of preparing a polyether by the preparation method as defined in any one of Claims 1 to 10 and a step of reacting the polyether and a polyisocyanate compound to obtain a prepolymer having an isocyanate group at the terminal.

13. A modified silicone polymer preparation method which comprises a step of preparing a prepolymer by the preparation method as defined in Claim 12 and a step of introducing a hydrolysable silyl group to the molecular terminal of the prepolymer.

14. The modified silicone polymer preparation method according to Claim 11 or 13, wherein the modified silicone polymer is a sealant.

Fig. 1

## Fig. 2

Fig. 3

## INTERNATIONAL SEARCH REPORT

| | International application No. |
|---|---|
| | PCT/JP2012/078388 |

A. CLASSIFICATION OF SUBJECT MATTER
*C08G65/28*(2006.01)i, *C08G65/336*(2006.01)i, *C09K3/10*(2006.01)i

According to International Patent Classification (IPC) or to both national classification and IPC

B. FIELDS SEARCHED

Minimum documentation searched (classification system followed by classification symbols)
C08G65/00-65/48, C09K3/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 05-247199 A (Asahi Glass Co., Ltd.), 24 September 1993 (24.09.1993), claims 1 to 2; paragraph [0022] (Family: none) | 1-14 |
| Y | JP 06-200012 A (Mitsui Toatsu Chemicals, Inc.), 19 July 1994 (19.07.1994), claims 1 to 2; paragraph [0002] (Family: none) | 1-14 |
| Y | WO 2011/111797 A1 (Asahi Glass Co., Ltd.), 15 September 2011 (15.09.2011), claims 1 to 7 & CN 102782046 A    & TW 201139485 A | 11-14 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22 January, 2013 (22.01.13) | 05 February, 2013 (05.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/078388

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-342361 A  (Nippon Shokubai Co., Ltd.),<br>03 December 2003 (03.12.2003),<br>paragraph [0037]<br>& US 2003/0004377 A1     & EP 1258502 A2<br>& EP 2333002 A1 | 1-14 |
| A | JP 2004-231883 A  (Nippon Zeon Co., Ltd.),<br>19 August 2004 (19.08.2004),<br>claims 1 to 6<br>(Family: none) | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5247199 A
- JP 2946580 B
- JP 2011241910 A